Europäisches Patentamt

European Patent Office

Office européen des brevets

(11) Publication number: **0 130 455
B1**

(12) **EUROPEAN PATENT SPECIFICATION**

(45) Date of publication of patent specification: **10.05.89**

(51) Int. Cl.⁴: **G 01 N 21/90**

(21) Application number: **84106980.0**

(22) Date of filing: **19.06.84**

(54) **Process for detecting resident air in a liquid charged receptacle and apparatus therefor.**

(30) Priority: **24.06.83 JP 113823/83**

(43) Date of publication of application:
**09.01.85 Bulletin 85/02**

(45) Publication of the grant of the patent:
**10.05.89 Bulletin 89/19**

(84) Designated Contracting States:
**AT BE CH DE FR GB IT LI NL SE**

(56) References cited:
**EP-A-0 087 484
GB-A-2 001 751
US-A-3 496 369
US-A-4 274 745**

(73) Proprietor: **Eisai Co., Ltd.
6-10, Koishikawa 4-chome Bunkyo-ku
Tokyo 112 (JP)**

(72) Inventor: **Tagaya, Ryosaku
No. 79-1, Gedoji-cho
Isesake-shi Gunma-ken (JP)**
Inventor: **Kojima, Osamu
No. 1108 Tateishi
Fujioka-shi Gunma-ken (JP)**
Inventor: **Sonobe, Yasuo
No. 462-5, Oaza-Komoda Misato-mura
Kodama-gun Saitama-ken (JP)**

(74) Representative: **Patentanwälte Schulze Horn
und Hoffmeister
Goldstrasse 36
D-4400 Münster (DE)**

EP 0 130 455 B1

Courier Press, Leamington Spa, England.

**Description**

This invention relates to a method of detecting non-liquid phases having scattering or reflecting properties within a liquid, said liquid contained in a transparent receptacle in a rotation adapted form, said method comprising rotating said receptacle at high rotational speed about the axis of the receptacle to cause the contents to rotate, and detecting non-liquid phases by an incident light beam reflected at the optical boundaries of the non-liquid phase against the liquid.

In the field of medical treatment transparent receptacles with an anesthetic or the like are used. As the liquid in the receptacle has to be pure, all the receptacles containing non-liquid phases, like foreign particles, have to be eliminated. However, it is difficult to detect these particles by the eye from the outside.

In US—A 3,496,369 a method is described by which non-liquid phases like dust, glass splinters or fibers can be detected within a liquid contained in a totally transparent receptacle. The receptacle is rotatably supported by two receptacle supporters fixing the receptacle at the bottom and the upper end. The two supporters establish the rotation axis of the receptacle. If the receptacle is rotated at high rotational speed, the boundaries of the non-liquid phases an the liquid are detected because of the different optical qualities of reflecting light.

During the rotation the non-liquid phases, namely the foreign particles, concentrate in the vicinity of the center of the receptacle. A sample inspecting apparatus for inspection the liquid has a portion capable of transmitting a light beam. The beam is reflected by the non-liquid phases indicating that foreign particle are inside of the receptacle.

The disadvantage of the method described in US—A 3,496,369 is that no gas or air can be detected. The gas or air is essential for such receptacles in order to compensate the pressure build up by the volume extension of the liquid. Therefore the object of the known method is to detect foreign solid particles and not to detect air cavities. Although the boundary surface of air and liquid will be deformed by rotation, the generation of bubbles in the receptacles has to be prevented because the generated bubbles scatter light from the light source, resulting in a cause of an error signal. Bubbles which may occur are understood as a disturbance or nuisance.

In the GB—A 2,001,751 a method is described to detect non-liquid phases, namely solid particles, being present in a liquid contained in a totally transparent receptacle made of glass. The receptacle to be inspected is rotated at high speed and is then brought to a standstill quickly. By this treatment the solid particles are suspended and swirl together with the liquid. The receptacle is then illuminated and the light which passes through the liquid is received by an optical detector. Judgement as to whether to accept or reject the receptacle is made according to the degree of decrease in light received in comparison with the reference value.

The disadvantage of the method described in the GB—A 2,001,751 is comparable to the problem described in the US—A 3,496,369. Both methods can only be used for the identification of solid particles. To avoid error signals the surface boundary of liquid and air has to be stable and coherent. The acceleration and the slow down of the rotation must be so moderate and careful that the surface boundary is not disturbed.

All the known methods are not applicable to receptacles containing residual air and being covered at the mouth portion by an opaque seal member.

It is the object of the invention to provide a detecting method which is capable of identification of residual air as the non-liquid phase in a liquid-filled receptacle having an opaque sealing member disposed over the upper end of the receptacle, said sealing member covering the boundary surface between air and liquid in an erect and still position of the receptacle and to decide whether the detected amount of air or gas exceeds a tolerable range. If the amount is higher than tolerable the receptacle shall be eliminated. The method should be efficient and practicable in known apparatuses.

The object of the invention is achieved by a method as defined in the first paragraph of the description characterised by application to the above object and by the steps of rotating the receptacle at a revolving speed of about 5,000— 6,000 rpm causing the boundary surface to deform and abruptly stopping the rotation of the receptacle, thereby causing the residual air to form an air bubble appearing below said sealing member, and detecting the bubble below the sealing member by the reflection of said incident light beam from said bubble.

In the receptacle rotating at said speed, the air cavity forms a paraboloid of revolution. The lower end of the air cavity is located deeper than the seal member. After the rotation has been stopped a bubble-shaped air cavity is created, descending beyond the lower rim of the sealing member. A light beam inciding on said air is reflected from the phase boundaries and is received by a receiver. Thus, the presence of resident air and the approximate amount of air may be detected.

The invention is described referring to an apparatus shown in the drawing. The figures represent in detail:

Fig. 1A is a longitudinal front elevation of a liquid filled receptacle with resident air;

Fig. 1B is a plane view of the receptacle of Fig. 1A;

Fig. 2A is a plane view of the receptacle of Fig. 1A illustrating one mode of the detection of residual air;

Fig. 2B is a plane view of the liquid receptacle illustrating another mode of detecting of residual air in the same situation as Fig. 2A;

Fig. 3 is a schematic plane view illustrating an apparatus for applying the inventive detecting method;

Fig. 4 is an enlarged detail and front view illustrating the positional relationship between the apparatus according to Fig. 3 and receptacle driving member;

Fig. 5 is a detail of Fig. 3 illustrating the relationship between the air detecting apparatus according to this invention and the receptacle in Fig. 3;

Fig. 6 is a right side view of Fig. 5.

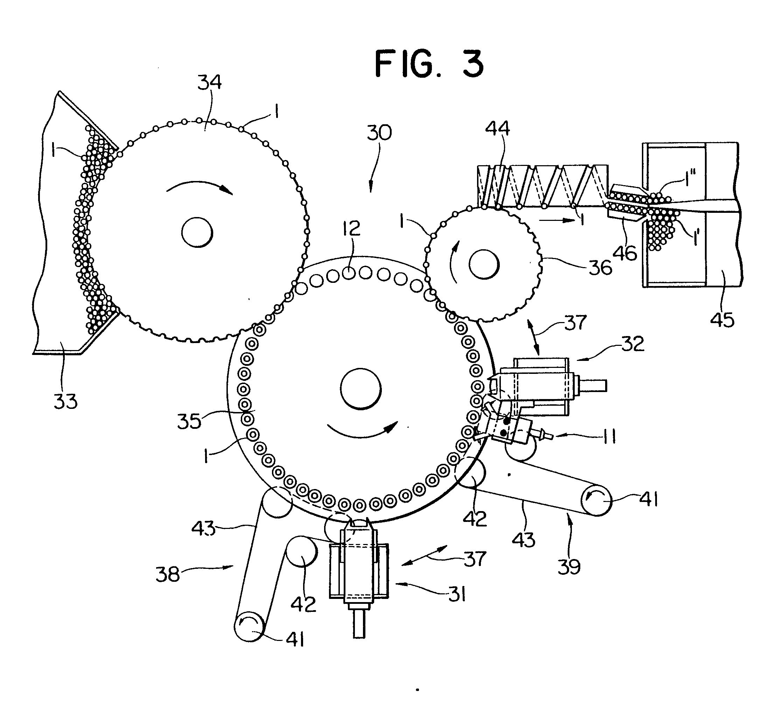

Fig. 3 schematically illustrates an apparatus 30 for discerning receptacles 1, 1', 1'' comprising an air detecting apparatus 11 together with different first and second apparatusses 31 and 32 for detecting a badly filled receptacle. A supply hopper 33, a first turntable 34 arranged to border the supply hopper 33 and to rotate continuously at a fixed speed, a second turntable 35 disposed adjacent to the table 34 and designed to rotate simultaneously therewith, and a third turntable 36 disposed adjacent to the table 35 and arranged to rotate simultaneously therewith, are parts of said apparatus 30.

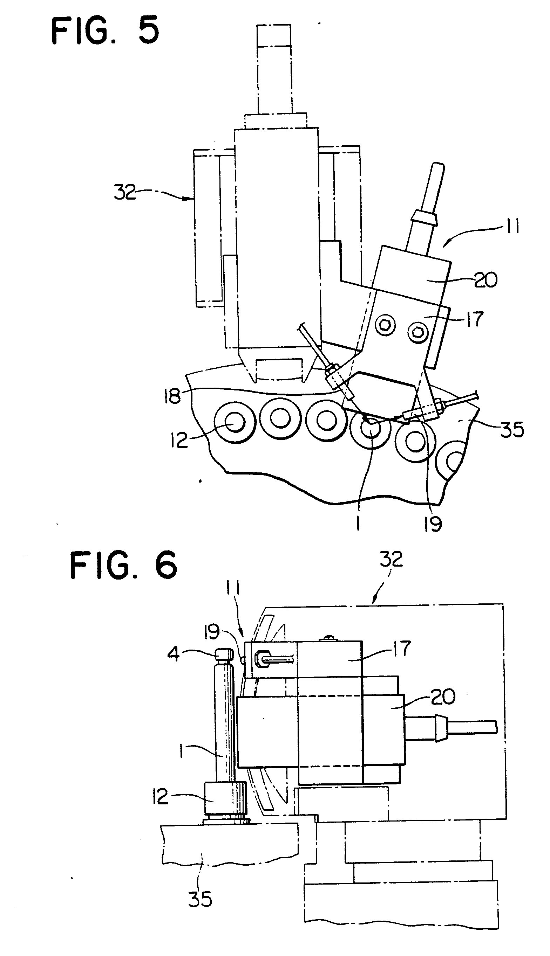

At the periphery of the turntable 35 the first detecting apparatus 31 and the second detecting apparatus 32 are provided, so as to make a reciprocating movement in the circular direction shown by the arrows 37. On one side of each of these apparatusses 31 and 32 there is provided a first driving apparatus 38 and a second driving apparatus 39, respectively. These driving apparatusses 38 and 39 are similar in construction. Each includes a driving pulley 41 rotated by a motor 40 which is illustrated in Fig. 6 alone, further driven pulleys 42 and a belt 43 passing around these pulleys.

Reference numeral 44 denotes a screw conveyor for discharge 45 denotes a conveyor of discharge, respectively. Between both conveyors a selecting member 46 is provided oscillating vertically in Fig. 3.

The receptacles 1 are transferred from the hopper successively through turntables 34, 35 and 36, and are discharged on the conveyor 45 via the conveyor 44 from the turntable 36. During the transfer, the presence of foreign particles contained in the receptacle is detected by using the first detecting apparatus 31. In case foreign particles are detected, a deferred signal is given to the selecting member 46. Upon receiving said signal, the selecting member 46 oscillates downward in Fig. 3 so as to discharge said receptacle 1 containing foreign particles on the conveyor 45 as inferior (1''). In case the receptacle 1 is regarded as being in order, the selecting member oscillates upward so as to discharge said receptacle 1 as acceptable (1'').

Generally the same test is performed by the second detecting apparatus 32. During the tests the receptacle 1 is rotated at high speed by the action of belts 43 of the first driving apparatus 38 and the second driving apparatus 39.

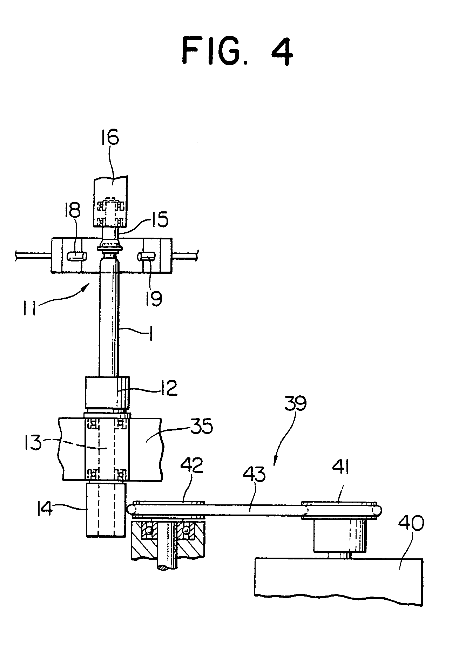

The mechanism is shown in Fig. 4 together with an air detecting apparatus 11. Said detecting apparatus 11 will be explained referring to Fig. 4 to Fig. 6.

Fig. 4 shows the mechanism for rotating the receptacle 1 on the turntable 35 at high rotational speed. Several rotary discs 12 are disposed at the outer periphery of the turntable 35, at regular intervals in the circular direction. Its rotary shaft 13 penetrates the table, is thereby supported rotatably, extends downward and bears at its lower end the friction roller 14. This roller 14 is friction connected with the belt 43. Above the rotary disc 12 a rotary pad 15, which thrusts the top of the receptacle 1 downward under pressure, is attached to the table 35 through a supporting bar 16.

Fig. 5 and Fig. 6 show the detecting apparatus 11 attached to the second detecting apparatus 32 though a bracket 17. This detecting apparatus 11, comprises a projector 18 and a ray receiver 19 which are arranged below and adjacent to a sealing member 4 of the receptacle 1, being at the same level. When a ray coming from the projector 18 hits on the surface boundary of the air 5' or 5'' and reflects therefrom, the reflected light may be received by the ray receiver 19.

Reference numeral 20 denotes an electrical capacitance type proximity switch. This switch 20 is designed to detect the case where the receptacle 1 is empty.

In detecting the air in the receptacle by means of the above mentioned apparatus, the receptacle 1 turning on the turntable 35 is rotated at a high rotational speed of 5,000—6,000 rpm by means of the second driving apparatus 39. When a ray is projected on the rotating receptacle 1 from the projector 18 the following may occur:

1) In case no air is resident in the receptacle 1, no phase boundary is present at the portion of the receptacle 1 where the ray travels. The ray is not reflected and passes through, consequently the ray receiver 19 does not receive a reflected ray.

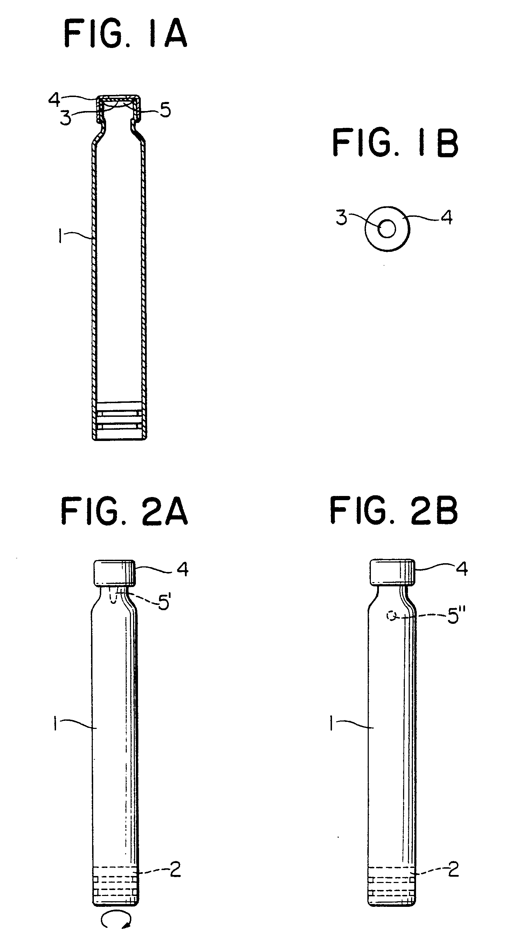

2) In case air is resident in the receptacle 1 and its amount is relatively high, the boundary surface of the residual air 5 is deformed as shown in Fig. 2A. A ray travelling as shown with an arrow in Fig. 5 is reflected by the air 5'. The reflected ray is received by the ray receiver 19, whereby the presence of a relatively high amount of residual air can be detected.

3) In case the amount of residual air in the receptacle 1 is relatively small, that boundary may not be detected like the air 5' during the turning motion. But the residual air 5'' forms a bubble at the time of stopping. Said bubble descends, the projected ray is reflected from said bubble 5'' and the reflected ray is received by the ray receiver 19. Therefore, the residual air in the receptacle 1 is to be detected even if its amount is relatively small.

It can be seen from the above that in case of (2) and (3) air detected, but the case of (3) is normally regarded as acceptable, because the amount of residual air is small. Accordingly, the case (2) must be regarded as "inferior", but the cases (1) and (3) will be acceptable.

If therefore the detecting apparatus 11 is designed to send its detection signal to a selecting member 46 only in the case of (2), it will be possible to select only receptacles 1 under the conditions as shown in Fig. 2A as inferior and carry them on the conveyor for discharge. In the aforesaid embodiment, the air detecting apparatus 11 is placed side by side with the second detecting apparatus 32. Said air detecting apparatus 11 may be used independently having no connection with this detecting apparatus or with the first detecting apparatus 31.

### Claim

A method of detecting non-liquid phases having scattering or reflecting properties within a liquid, said liquid contained in a transparent receptacle (1) in a rotation adapted form, said method comprising rotating said receptacle at high rotational speed about the axis of the receptacle to cause the contents to rotate, and detecting non-liquid phases by an incident light beam reflected at the optical boundaries of the non-liquid phase against the liquid, characterized by application of the method to a liquid-filled receptacle (1) containing residual air (5) as the non-liquid phase, and having an opaque sealing member (4) disposed over the upper end of the receptacle (1), said sealing member (4) covering the boundary surface between air and liquid in an erect and still position of the receptacle, by the steps of rotating the receptacle (4) at a revolving speed of about 5,000—6,000 rpm causing the boundary surface to deform, and abruptly stopping the rotation of the receptacle (4), thereby causing the residual air to form an air bubble (5'') appearing below said sealing member, and detecting the bubble (5'') below the sealing member by the reflection of said incident light beam from said bubble.

### Patentanspruch

Verfahren zum Nachweis von nicht-flüssigen Phasen, die innerhalb einer Flüssigkeit lichtstreuende oder -reflektierende Eigenschaften besitzen, wobei die genannte Flüssigkeit in einem durchsichtigen, zum Rotieren geeigneten Gefäß (1) enthalten ist, das Verfahren bestehend aus der Rotation des Gefäßes bei hoher Drehgeschwindigkeit um die eigene Achse, um den Inhalt zum Rotieren zu bringen sowie aus dem Nachweis nicht-flüssiger Phasen mittels eines einfallenden Lichtstrahls, der an den optischen Grenzen der nicht-flüssigen Phase der Flüssigkeit reflektiert wird, gekennzeichnet durch die Anwendung des Verfahrens auf ein mit Flüssigkeit gefülltes Gefäß (1), das Restluft (5) als nicht-flüssige Phase enthält, und einen undurchsichtigen Verschlußteil (4) über der Öffnung am oberen Ende des Gefäßes (1) besitzt, der die Grenzfläche zwischen der Luft und der Flüssigkeit abdeckt, wenn das Gefäß in einer aufrechten Stellung ruht, weiter gekennzeichnet durch die Schritte: Rotation des Gefäßes (4) bei einer Drehgeschwindigkeit von etwa 5.000 bis 6.000 Umdrehungen pro Minute, die eine Verformung der Grenzfläche hervorruft, sowie abruptes Anhalten der Gefäßrotation, wobei die Restluft unterhalb des Verschlußteils eine Luftblase (5'') erzeugt, und Nachweis der Luftblase (5'') unterhalb des Verschlußteils durch Reflektion des genannten einfallenden Lichtstrahls an der genannten Luftblase.

### Revendication

Procédé de détection de phases non liquides ayant des propriétés de dispersion ou de réflexion à l'intérieur d'un liquide, ce liquide étant contenu dans un réceptacle transparent (1) dont la forme est adaptée à la rotation, ledit procédé comprenant la mise en rotation dudit réceptacle à une grande vitesse de rotation autour de l'axe du réceptacle pour provoquer la rotation du contenu, et la détection des phases non liquides par un faisceau de lumière incidente réfléchi aux surfaces de séparation optique de la phase non liquide par rapport au liquide, caractérisé par l'application du procédé à un réceptacle rempli de liquide (1) contenant de l'air résiduel (5) comme phase non liquide et comportant un élément de fermeture opaque (4) placé sur l'extrémité supérieure du réceptacle (1), ledit élément de fermeture (4) recouvrant la surface de séparation entre l'air et le liquide en position debout et immobile du réceptacle, et par les opérations de mise en rotation du réceptacle (1) à une vitesse de rotation de 5 000 à 6 000 t/mn environ, qui provoque la déformation de la surface de séparation, et d'arrêt brusque de la rotation du réceptacle (1), de sorte que l'air résiduel forme une bulle d'air (5'') apparaissant au-dessous dudit élément de fermeture, et de détection de la bulle (5'') au-dessous de l'élément de fermeture du fait de la réflexion dudit faisceau de lumière incidente par ladite bulle.

EP 0 130 455 B1

FIG. 1A

FIG. 1B

FIG. 2A

FIG. 2B

1

# FIG. 3

EP 0 130 455 B1

# FIG. 4

# FIG. 5

# FIG. 6